# EUROPEAN PATENT APPLICATION

(11) **EP 4 591 996 A1**
(43) Date of publication of application: **30.07.2025**
(21) Application number: 23882530.1
(22) Date of filing: 19.10.2023
(51) Int. Cl.: B05C 19/04, B05B 3/02, B65G 65/32, A61F 13/15

(54) **PARTICULATE MATTER DISPERSING DEVICE**

(30) Priority: 26.10.2022 JP 2022171609
(71) Applicant: Zuiko Corporation, Ibaraki-shi, Osaka 5670082 (JP)
(72) Inventor: SHIMADA, Takahiro, Ibaraki-shi, Osaka 567-0082 (JP)
(74) Representative: Hasegawa, Kan
(86) International application number: PCT/JP2023/037871
(87) International publication number: WO 2024/090325

(57) **Abstract**

Provided is a particle dispersing apparatus that can prevent leakage of particles from a rotary body. The particle dispersing apparatus (1) includes: a rotary body (6) having a cylindrical portion (6a) that is supported by a support member (5) rotatably around a rotational axis (C1) extending in a horizontal direction, and a dispersion hole (6b) extending through the cylindrical portion in a hole formation region (R1) that is a part of the cylindrical portion (6a) in an axial direction along the rotational axis (C1); and a particle introducing member (7) that has a housing portion (7a) having a housing space (SP) for housing particles and an opening part (7b) formed in a lower end of the housing portion (7a) to introduce the particles into the cylindrical portion (6a), and is attached to the support member (5) such that the opening part (7b) is located in the cylindrical portion (6a), and the opening part (7b) of the housing portion (7a) has an edge portion that comes into contact with a region of an inner circumferential surface of the cylindrical portion (6a) below the rotational axis (C1) while allowing the region of the inner circumferential surface of the cylindrical portion to slide against the edge portion, the edge portion overlapping with the hole formation region (R1) in the axial direction entirely so as to confine the particles in cooperation with the cylindrical portion (6a).

## Description

### Technical Field

The present invention relates to a particle dispersing apparatus.

### Background Art

There has been conventionally known a particle dispersing apparatus that disperses particles to a dispersion object. For example, Patent Literature 1 discloses superabsorbent polymer particulate dispersing means for dispersing superabsorbent polymer particulates.

The superabsorbent polymer particulate dispersing means disclosed in Patent Literature 1 have a rotary drum configured to be rotatable around a rotational axis extending in a certain direction and surrounding the rotational axis, and a hopper connected to the rotary drum to supply superabsorbent polymer particulates into the rotary drum. The rotary drum has a projection hole for dispersing the superabsorbent polymer particulates supplied from the hopper.

Patent Literature 1 does not disclose a specific structure for connecting the rotary drum and the hopper. The rotary drum is required to rotate to project the superabsorbent polymer particulates inside by a centrifugal force, and the hopper is required to be fixed so as to introduce the superabsorbent polymer particulates into the rotary drum under their own weight. Therefore, a conceivable connection between the rotary drum and the hopper is as follows.

Specifically, a lid member is attached to an opening end of the rotary drum in an axial direction of the rotary drum such that the opening end is slidable against the lid member, in order to confine the superabsorbent polymer particulates in the rotary drum. The lid member is fixed to a part of the superabsorbent polymer particulate dispersing means which is fixed to an installation place thereof, and the rotary drum is attached to the lid member so as to allow supply of the superabsorbent polymer particulates into the rotary drum through an insertion hole formed in the lid member. These enable supplying the superabsorbent polymer particulates from the hopper into the rotary drum while allowing the rotary drum to rotate relative to the hopper.

However, in the case where the rotary drum and the hopper are connected with each other as described above, the lid member is required to come into contact with an entire circular end surface of the rotary drum in the axial direction so as to allow the rotary drum to slide against and rotate relative to the lid member while preventing leakage of superabsorbent polymer. Therefore, wear of at least one of the rotary drum and the lid member may cause leakage of the superabsorbent polymer particulates.

### Citation List

### Patent Literature

Patent Literature 1: Japanese Patent No. 4790281

### Summary of Invention

An object of the present invention is to provide a particle dispersing apparatus that can prevent leakage of particles from a rotary body.

To solve the above-mentioned problem, provided is a first invention directed to a particle dispersing apparatus to disperse particles to a dispersion object, including: a support member; a rotary body having a cylindrical portion that is supported by the support member rotatably around a rotational axis extending in a horizontal direction and surrounds the rotational axis, and a dispersion hole extending through the cylindrical portion in a hole formation region that is a part of the cylindrical portion in an axial direction along the rotational axis; and a particle introducing member that has a housing portion having a housing space for housing the particles and an opening part formed in a lower end of the housing portion to introduce the particles into the cylindrical portion, and is attached to the support member such that the opening part is located in the cylindrical portion, and in the particle dispersing apparatus, the opening part of the housing portion has an edge portion that comes into contact with a region of an inner circumferential surface of the cylindrical portion below the rotational axis while allowing the region of the inner circumferential surface of the cylindrical portion to slide against the edge portion, the edge portion overlapping with the hole formation region in the axial direction entirely so as to confine the particles in cooperation with the cylindrical portion.

The present invention enables provision of a particle dispersing apparatus that can prevent leakage of particles from a rotary body.

### Brief Description of Drawings

FIG. 1 is a front view of a particle dispersing apparatus, showing a whole structure thereof but excluding a part of a scraper and a return mechanism.
FIG. 2 is a sectional view taken along a line II-II in FIG. 1.
FIG. 3 is a sectional view taken along a line III-III in FIG. 2.
FIG. 4 is a front sectional view showing a shutter in FIG. 3 rotated to a closure position.

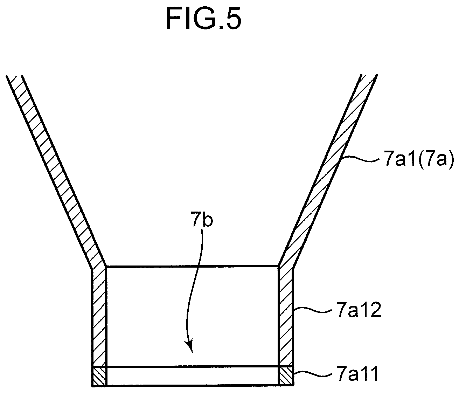

FIG. 5 is a sectional view taken along a line V-V in FIG. 3.
FIG. 6 is a net obtained by unfolding an inner circumferential surface of a cylindrical portion shown in FIG. 3, which shows a relationship between an opening part of a particle introducing member and a scraper.
FIG. 7 is an enlarged plan view of a mesh section of a conveying belt in FIG. 1.
FIG. 8 is a block diagram showing an electrical configuration of a dispersing apparatus body in FIG. 1.
FIG. 9 is a flowchart showing a shutter driving process to be executed by a controller in FIG. 8.
FIG. 10 is a flowchart showing a particle returning process to be executed by the controller in FIG. 8.

### Description of Embodiments

Hereinafter, an embodiment of the present invention will be described with reference to the accompanying drawings. It should be noted that the following embodiment illustrates one specific example of the present invention, and does not delimit the protection scope of the present invention.

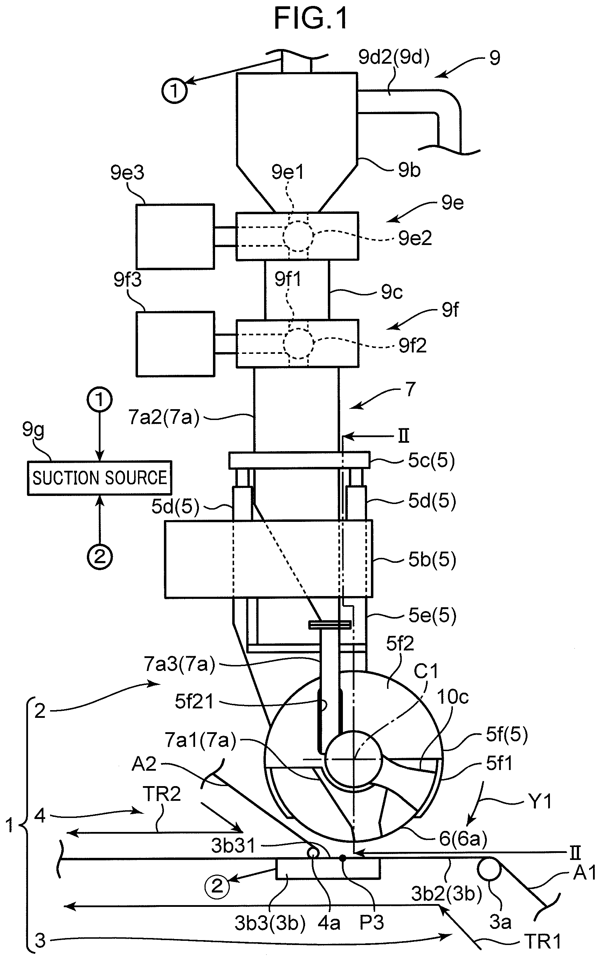

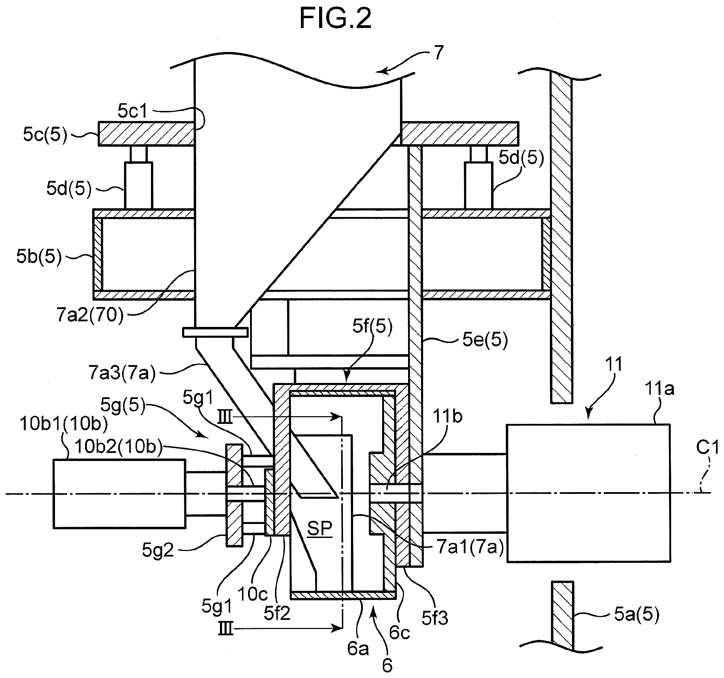

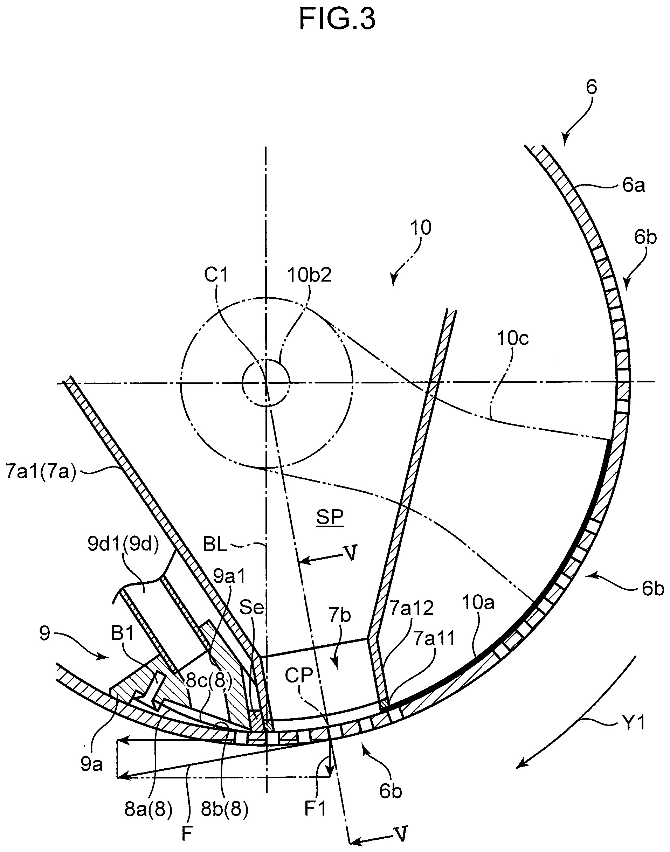

FIG. 1 is a front view of a particle dispersing apparatus, showing a whole structure thereof but excluding a part of a scraper and a return mechanism. FIG. 2 is a sectional view taken along a line II-II in FIG. 1. FIG. 3 is a sectional view taken along a line III-III in FIG. 2.

With reference to FIGS. 1 to 3, a particle dispersing apparatus 1 disperses particles to a dispersion object A1. For example, the dispersion object A1 is a continuous body of, e.g., absorbent units (obtained by placing an absorbent core on a nonwoven fabric) for use in a disposable wearable article. The particles include, e.g., particles of citric acid to be dispersed to a part for absorption of excretion of a wearer of the disposable wearable article.

The particle dispersing apparatus 1 includes a dispersing apparatus body 2 to disperse the particles to the dispersion object A1, a first conveying part 3 that is provided below the dispersing apparatus body 2 and continuously conveys the dispersion object A1, which has a strip shape, and a second conveying part 4 that continuously conveys a covering member A2 having a strip shape over the dispersion object A1 to cover the dispersion object A1 conveyed by the first conveying part 3.

The dispersing apparatus body 2 includes a support member 5 fixed to a certain installation place, a rotary body 6 supported by the support member 5 rotatably around a rotational axis C1 extending in a horizontal direction, a particle introducing member 7 to introduce the particles into the rotary body 6, a scraper (removal member) 8 provided inside the rotary body 6 to remove particles remaining in the rotary body 6, a return mechanism 9 that returns the particles removed by the scraper 8 to the particle introducing member 7, a dispersion amount adjusting mechanism 10 to adjust an amount of particles to be dispersed from the rotary body 6, and a rotary body driving motor 11 that drives the rotary body 6 rotatably.

With reference to FIGS. 1 and 2, the support member 5 has a vertical plate 5a standing in the certain installation place, and a support mechanism that is provided on a front surface of the vertical plate 5a to support the rotary body 6, the particle introducing member 7, the scraper 8, the return mechanism 9, the dispersion amount adjusting mechanism 10, and the rotary body driving motor 11. The support mechanism includes a forward protruding portion 5b that protrudes forward from the front surface of the vertical plate 5a, a load receiving plate 5c above the forward protruding portion 5b, a weight detector 5d between the forward protruding portion 5b and the load receiving plate 5c, a downward extending plate 5e that extends downward from the load receiving plate 5c, parallel to the vertical plate 5a, a cover 5f that is fixed to a front surface of the downward extending plate 5e and covers the rotary body 6, and an adjusting mechanism attachment portion 5g for attaching the dispersion amount adjusting mechanism 10 to a front surface of the cover 5f.

The forward protruding portion 5b has a through hole 5b1 extending therethrough in an up-down direction to allow the particle introducing member 7 and the downward extending plate 5e to pass through the forward protruding portion.

The load receiving plate 5c has a through hole 5c1 extending therethrough in the up-down direction to allow the particle introducing member 7 to pass through the load receiving plate, and is fixed to the particle introducing member 7 placed in the through hole 5c1. Thus, the load receiving plate 5c receives a load of the particle introducing member 7 and the return mechanism 9 fixed to an upper portion thereof. The load receiving plate 5c additionally receives a load of the rotary body 6, the scraper 8, the dispersion amount adjusting mechanism 10, and the rotary body driving motor 11 via the downward extending plate 5e and the cover 5f. Therefore, a detection by the weight detector 5d of a decrease in a load received by the load receiving plate 5c during a certain period results in a determination of a dispersion amount of particles during the certain period. The weight detector 5d has, e.g., a load cell.

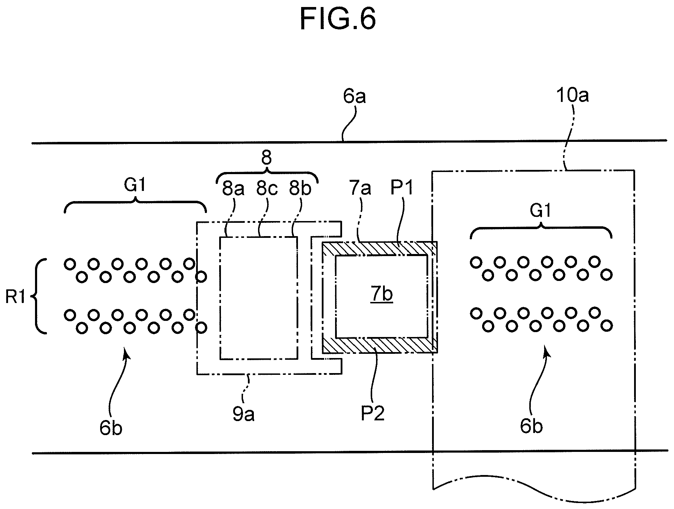

The rotary body 6 has a cylindrical portion 6a that is supported by the downward extending plate 5e rotatably around the rotational axis C1 and surrounds the rotational axis C1, a dispersion hole 6b extending through the cylindrical portion 6a in a hole formation region R1 (see FIG. 6) that is a part of the cylindrical portion 6a in an axial direction along the rotational axis C1, and a closing plate 6c fixed to a rear end of the cylindrical portion 6a to close a rearward opening of the cylindrical portion 6a. As shown in FIG. 6, the hole formation region R1 has a plurality of the dispersion holes 6b. The cylindrical portion 6a has a plurality of groups G1, each of which is constituted by the dispersion holes 6b, the groups G1 being formed on the cylindrical portion 6a intermittently in a circumferential direction around the rotational axis C1.

As shown in FIGS. 1 and 2, a partial region of an outer circumferential surface of the cylindrical portion 6a in the circumferential direction is covered by an outer circumference covering part 5f1 of the cover 5f described above. Specifically, the outer circumference covering part 5f1 covers a region of the outer circumferential surface of the cylindrical portion 6a above a position in the up-down direction between the rotational axis C1 and an opening part 7b of the particle introducing member 7 described later. The outer circumference covering part 5f1 may cover the outer circumferential surface of the cylindrical portion 6a entirely except a region facing the opening part 7b in the circumferential direction around the rotational axis C1. The cylindrical portion 6a has a forward opening, which is covered by a front covering part 5f2 of the cover 5f described above from the front. The front covering part 5f2 covers an area in front of the cylindrical portion 6a, the area including an area above a position between the rotational axis C1 and a lower end of the cylindrical portion 6a as shown in FIG. 1 and an area overlapping with a shutter driving motor 10b described later in a front-rear direction. The closing plate 6c of the rotary body 6 is covered from the rear by a rear covering plate 5f3 of the cover 5f. The rear covering plate 5f3 covers an area of the closing plate 6c, the area including an area above a position between the rotational axis C1 and a lower end of the closing plate 6c and an area overlapping with the rotary body driving motor 11 described later in the front-rear direction.

As shown in FIG. 2, the rotary body driving motor 11 has a motor body 11a fixed to the downward extending plate 5e, and a drive shaft 11b extending forward from the motor body 11a. The drive shaft 11b extends forward through insertion holes formed in the downward extending plate 5e and the rear covering plate 5f3 so as to rotate around the rotational axis C1 with respect to the downward extending plate 5e and the rear covering plate 5f3. The drive shaft 11b has a front end fixed to the closing plate 6c of the rotary body 6. Thus, a rotation of the drive shaft 11b with respect to the motor body 11a causes the rotary body 6 to rotate relative to the support member 5. The drive shaft 11b rotates in a rotational direction Y1 shown by an arrow in FIG. 3 to disperse the particles.

With reference to FIGS. 1 to 3, the particle introducing member 7 has a housing portion 7a having a housing space SP for housing the particles and the opening part 7b formed in a lower end of the housing portion 7a to introduce the particles into the cylindrical portion 6a, and is attached to the support member 5 (the load receiving plate 5c) such that the opening part 7b is located in the cylindrical portion 6a. The opening part 7b of the housing portion 7a has an edge portion that comes into contact with a region of an inner circumferential surface of the cylindrical portion 6a below the rotational axis C1 while allowing the region of the inner circumferential surface of the cylindrical portion to slide against the edge portion, the edge portion overlapping with the hole formation region R1 in the axial direction (the front-rear direction) entirely so as to confine the particles in cooperation with the cylindrical portion 6a (see FIG. 6).

In order that the edge portion of the opening part 7b "confines the particles in cooperation with the cylindrical portion 6a", two sections P1, P2 (hereinafter, referred to as axial directional opposite side sections) of the edge portion of the opening part 7b (see hatched sections in FIG. 6), which are across the hole formation region R1 in the axial direction, are required to come into contact with the inner circumferential surface of the cylindrical portion 6a such that the inner circumferential surface is slidable so as to prevent leakage of the particles in the axial direction. On the other hand, even if particles leak from a region between the axial directional opposite side sections P1, P2 of the edge portion of the opening part 7b on the inner circumferential surface of the cylindrical portion 6a, the particles are dispersed from the dispersion holes due to a centrifugal force caused by the rotation of the cylindrical portion 6a, thus resulting in no substantial leakage of the particles. In other words, the axial directional opposite side sections P1, P2 of the edge portion of the opening part 7b are the most important to "confine the particles in cooperation with the cylindrical portion 6a". FIG. 6 is a net of a part of the cylindrical portion 6a in the circumferential direction, which is obtained by cutting the part in the circumferential direction out of the cylindrical portion 6a and unfolding it.

A specific structure of the housing portion 7a will be described below with reference to FIGS. 1 to 3.

The housing portion 7a has an inner housing part 7a1 that has the opening part 7b and is located in the cylindrical portion 6a, an outer housing part 7a2 that houses the particles and is located outside the cylindrical portion 6a, and a connecting part 7a3 that connects the inner housing part 7a1 and the outer housing part 7a2 to introduce the particles from the outer housing part 7a2 to the inner housing part 7a1.

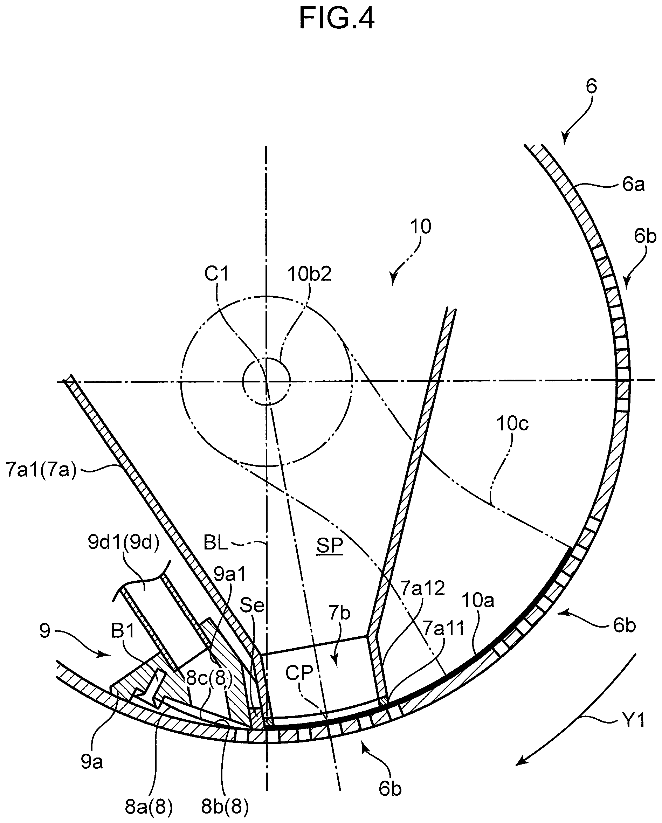

As shown in FIGS. 3 and 4, the inner housing part 7a1 has a contact part 7a11 that comes into contact with the inner circumferential surface of the cylindrical portion 6a, and an attached part 7a12 that is attached to the contact part 7a11, the attached part and the inner circumferential surface of the cylindrical portion 6a sandwiching the contact part 7a11. The attached part 7a12 is a container having a downward opening and tapering from up to down. The contact part 7a11 is attached to the edge portion of the opening of the attached part 7a12. In other words, the edge portion of the opening part 7b is formed by the contact part 7a11 and the attached part 7a12. The contact part 7a11 has an elasticity higher than an elasticity of the attached part 7a12. For example, the attached part 7a12 is made of, e.g., metal, and the contact part 7a11 is made of, e.g., felt. The edge portion of the opening in the attached part 7a12 has an arc front shape (see FIG. 3) and a side shape (see FIG. 4) along the inner circumferential surface of the cylindrical portion 6a. In the embodiment, the opening of the attached part 7a12 has a rectangular shape (see FIG. 6).

As shown in FIG. 2, the outer housing part 7a2 is a container tapering from up to down. The outer housing part 7a2 has a lower end located above the cylindrical portion 6a and in front of the cylindrical portion 6a.

As shown in FIGS. 1 and 2, the connecting part 7a3 is a cylindrical member that connects an upper end of the inner housing part 7a1 (the attached part 7a12) and a lower end of the outer housing part 7a2. Specifically, the connecting part 7a3 has a lower end portion that extends through a through hole (reference sign omitted) formed in the upper end of the inner housing part 7a1 to be located in the inner housing part 7a1, a middle portion extending from the lower end portion (upward and forward) outside the inner housing part 7a1 through an insertion hole 5f21 (see FIG. 1) formed in the front covering part 5f2, and an upper end portion extending upward from the middle portion and connected to the outer housing part 7a2. Joints of the connecting part 7a3 with the inner housing part 7a1 and the outer housing part 7a2 are sealed to prevent leakage of the particles. In other words, the inner housing part 7a1, the outer housing part 7a2, and the connecting part 7a3 define the housing space SP.

The housing portion 7a is configured as described above, so that the particles are introduced from the outer housing part 7a2 into the cylindrical portion 6a and confined between the cylindrical portion 6a and the housing portion 7a, and then dispersed below the cylindrical portion 6a through the dispersion holes 6b formed in the cylindrical portion due to a rotation of the above-described rotary body driving motor 11 driven in the rotational direction Y1 shown by the arrow in FIG. 3.

The particle introducing member 7 (the inner housing part 7a1) is attached to the support member 5 (the front covering part 5f2) such that a central position CP of the opening part 7b is located upstream in the rotational direction Y1 of the rotary body 6 from a base line BL extending downward from the rotational axis C1, in a side view of the rotary body 6 along the rotational axis C1, i.e., from a viewpoint shown in FIG. 3. This enables the centrifugal force F applied to the particles by the rotary body 6 to include a force component F1 in a direction toward the dispersion object A1 located below the rotary body. Therefore, the particles can be placed on the dispersion object A1 reliably.

Further, as described above, the contact part 7a11, which is provided between the attached part 7a12 of the inner housing part 7a1 and the inner circumferential surface of the cylindrical portion 6a, has the elasticity higher than the elasticity of the attached part 7a12. Thus, the contact part 7a11 can deform elastically according to a condition of the inner circumferential surface of the cylindrical portion 6a, so that wear of the edge portion of the opening part 7b can be prevented. Additionally, in the embodiment, the dispersion amount adjusting mechanism 10 to adjust the dispersion amount of the particles by use of the elasticity of the contact part 7a11 is provided. A configuration of the dispersion amount adjusting mechanism 10 will be described below with reference to FIGS. 1 to 3.

The dispersion amount adjusting mechanism 10 includes a shutter 10a (an opening degree adjusting member: see FIG. 3) attached to the support member 5 (the front covering part 5f2) so as to move between the contact part 7a11 and the inner circumferential surface of the cylindrical portion 6a while causing the contact part 7a11 to deform elastically to adjust an opening degree of the opening part 7b of the particle introducing member 7.

As shown in FIG. 2, the dispersion amount adjusting mechanism 10 is attached to the adjusting mechanism attachment portion 5g provided on the front surface of the cover 5f. The adjusting mechanism attachment portion 5g includes a plurality of legs 5g1 extending forward from the front covering part 5f2 of the cover 5f, and a fixed plate 5g2 that is attached to front ends of the legs 5g1 and is away forward from the front covering part 5f2. The adjusting mechanism attachment portion 5g includes the shutter driving motor 10b attached to the fixed plate 5g2, an arm 10c fixed to a drive shaft 10b2 of the shutter driving motor 10b, and the shutter 10a fixed to a leading end of the arm 10c.

The shutter driving motor 10b includes a motor body 10b1 fixed to the fixed plate 5g2, and the drive shaft 10b2 that extends rearward from the motor body 10b1 through an insertion hole formed in the fixed plate 5g2 so as to rotate around the rotational axis C1 with respect to the fixed plate 5g2.

The arm 10c extends from the drive shaft 10b2 in a direction orthogonal to the rotational axis C1 and is positioned between the front covering part 5f2 and the fixed plate 5g2 (in front of the cylindrical portion 6a as shown in FIG. 2).

The shutter 10a extends rearward from the leading end of the arm 10c into the cylindrical portion 6a. Specifically, the shutter 10a has an outer circumferential surface (arc surface) having such a diameter corresponding to a diameter of the inner circumferential surface of the cylindrical portion 6a as to come into slidable contact with the inner circumferential surface of the cylindrical portion 6a, in a view along the rotational axis C1 in FIG. 3. The shutter 10a has such a thickness as to be insertable between the contact part 7a11 and the inner circumferential surface of the cylindrical portion 6a while causing the contact part 7a1 1 to deform elastically. As shown in FIG. 6, the shutter 10a is large enough to cover the opening part 7b in the axial direction along the rotational axis C1 and the circumferential direction around the rotational axis C1.

The dispersion amount adjusting mechanism 10 described above can adjust the opening degree of the opening part 7b of the particle introducing member 7 by driving the shutter driving motor 10b to rotate the shutter 10a around the rotational axis C1. Specifically, the shutter 10a is attached to the support member 5 rotatably between: a fully open position shown in FIG. 3 where the shutter is away from a position between the opening part 7b and the inner circumferential surface of the cylindrical portion 6a in the circumferential direction to completely uncover the opening part 7b as shown in FIG. 3; and a fully closed position shown in FIG. 4 where the shutter is interposed between the opening part 7b and the inner circumferential surface of the cylindrical portion 6a to completely cover the opening part 7b. In the embodiment, the fully open position is located upstream in the rotational direction Y1 (see FIG. 3) of the rotary body 6 from the fully closed position shown in FIG. 4. As shown in FIG. 3, the shutter 10a at the fully open position is located between the contact part 7a11 and the inner circumferential surface of the cylindrical portion 6a. Thus, the shutter 10a can be kept sandwiched between the contact part 7a11 and the inner circumferential surface of the cylindrical portion 6a, so that sliding resistance generated on the shutter 10a during its rotation from the fully open position toward the fully closed position can be reduced in comparison with a case where the shutter 10a is rotated to a position that corresponds to the fully open position but is away from the contact part 7a11.

With reference to FIG. 3, the particles in the housing space SP of the particle introducing member 7 are dispersed (projected) from the cylindrical portion 6a every time the groups G1 including the dispersion holes 6b (see FIG. 6) face the opening part 7b due to the rotation of the cylindrical portion 6a in the rotational direction Y1. However, some particles may adhere to the inner circumferential surface of the cylindrical portion 6a due to, e.g., static electricity and remain on the inner circumferential surface of the cylindrical portion 6a to reach a position downstream in the rotational direction Y1 from the particle introducing member 7. The dispersing apparatus body 2 is provided with the scraper 8 to remove such particles from the inner circumferential surface of the cylindrical portion 6a and the return mechanism 9 to return the removed particles to the particle introducing member 7.

As shown in FIG. 3, the scraper 8 is attached to the support member 5 so as to come into contact with a region of the inner circumferential surface of the cylindrical portion 6a located below the rotational axis C1 and downstream in the rotational direction Y1 of the rotary body 6 from the opening part 7b while allowing the region of the inner circumferential surface of the cylindrical portion to slide against the scraper to remove the particles adhering to and remaining on the inner circumferential surface of the cylindrical portion 6a. Specifically, as shown in FIG. 6, the scraper 8 has a width enough to come into contact with the hole formation region R1 in the inner circumferential surface of the cylindrical portion 6a entirely in the axial direction along the rotational axis C1. As shown in FIG. 3, the scraper 8 is a plate member that has a base end part 8a located away from the inner circumferential surface of the cylindrical portion 6a, a leading end part 8b pressed on the inner circumferential surface of the cylindrical portion 6a at a position upstream in the rotational direction Y1 from the base end part 8a, and a middle part 8c between the base end part 8a and the leading end part 8b, the middle part 8c being curved to store a biasing force that presses the leading end part 8b in a direction toward the inner circumferential surface of the cylindrical portion 6a. The scraper 8 may be directly attached to the support member 5; however, in the embodiment, the base end part 8a of the scraper 8 is attached to the support member 5 via the return mechanism 9 described later.

The return mechanism 9 has a suction source 9g, and uses a suction force by the suction source 9g to introduce the particles removed by the scraper 8 to the particle introducing member 7. Specifically, as shown in FIGS. 1 and 3, the return mechanism 9 includes a capturing member 9a that captures the particles removed by the scraper 8, a storing container 9b that is connected to the suction source 9g to draw by suction the particles captured by the capturing member 9a and stores the particles, and a delivering container 9c that is placed below the storing container 9b and above the particle introducing member 7 (the outer housing part 7a2) and delivers the particles from the storing container 9b to the particle introducing member 7. As shown in FIG. 6, the capturing member 9a has a width enough to overlap with the hole formation region R1 and the scraper 8 entirely in the axial direction along the rotational axis C1. The capturing member 9a has a length enough to overlap with the scraper 8 entirely in the circumferential direction around the rotational axis C1. The capturing member 9a has a surface facing the inner circumferential surface of the cylindrical portion 6a, to which the base end part 8a of the scraper 8 is fixed by a screw B1. Further, the capturing member 9a has a first opening that faces in a direction toward the inner circumferential surface of the cylindrical portion 6a and a second opening that faces in a direction opposite to the direction toward the inner circumferential surface of the cylindrical portion 6a and deviating from the housing portion 7a (the inner housing part 7a1), and has a through hole 9a1 extending between the first opening and the second opening. As shown in FIG. 3, a sealing member Se is interposed between and sandwiched by the capturing member 9a and the inner housing part 7a1 in the circumferential direction around the rotational axis C1. The sealing member Se can prevent the particles from being scattered from between the capturing member 9a and the inner housing part 7a1.

The return mechanism 9 has a connection pipe 9d that connects the capturing member 9a and the storing container 9b, a first valve mechanism 9e provided between the storing container 9b and the delivering container 9c, and a second valve mechanism 9f provided between the delivering container 9c and the particle introducing member 7 (the outer housing part 7a2). The connection pipe 9d has a lower end portion 9d1 that is attached to the capturing member 9a and communicates with the through hole 9a1, a middle portion (not shown) extending from the lower end portion 9d1 outside the cylindrical portion 6a through a space below the front covering part 5f2 (see FIG. 1), and an upper end portion 9d2 that extends upward from the middle portion and is fixed to a side surface of the storing container 9b as shown in FIG. 1. The storing container 9b is connected to the suction source 9g via a pipe extending upward from the storing container 9b. The first valve mechanism 9e has a first passage 9e1 that connects the storing container 9b and the delivering container 9c, a first valve 9e2 to open and close the first passage 9e1, and a first valve driving motor 9e3 to drive the first valve 9e2 to open and close. The second valve mechanism 9f has a second passage 9f1 that connects the delivering container 9c and the particle introducing member 7, a second valve 9f2 to open and close the second passage 9f1, and a second valve driving motor 9f3 to drive the second valve 9f2 to open and close. In the embodiment, the valves 9e2, 9f2 are composed of butterfly valves rotatably driven by the valve driving motors 9e3, 9f3.

The return mechanism 9 returns the particles to the particle introducing member 7 while always keeping the storing container 9b under negative pressure, which will be described later. Specifically, when the particles are drawn by suction, the first valve 9e2 is opened and the second valve 9f2 is closed, so that the particles are drawn by suction into the delivering container 9c. When the particles are delivered to the particle introducing member 7, the first valve 9e2 is closed and the second valve 9f2 is opened, so that the particles in the delivering container 9c are delivered under their own weight to the particle introducing member 7 while the particles are drawn by suction into the storing container 9b.

A configuration of the first conveying part 3 that conveys the dispersion object A1 to hold the particles dispersed by the dispersing apparatus body 2 will be described below with reference to FIG. 1. The first conveying part 3 conveys the dispersion object A1 along a first conveying path TR1 set below the cylindrical portion 6a. The first conveying path TR1 includes an inclined part where the dispersion object A1 is conveyed diagonally upward from a position below the cylindrical portion 6a toward the cylindrical portion 6a, and a horizontal part where the dispersion object A1 is conveyed substantially horizontally from the inclined part along the rotational direction Y1 of the cylindrical portion 6a (see FIG. 3). Specifically, the first conveying part 3 has a plurality of conveying rollers including a conveying roller 3a provided at a boundary between the inclined part and the horizontal part, and a suction conveying mechanism 3b that conveys the dispersion object A1 while applying the suction force to the dispersion object A1 in a certain region of the first conveying path TR1. The certain region will be described later.

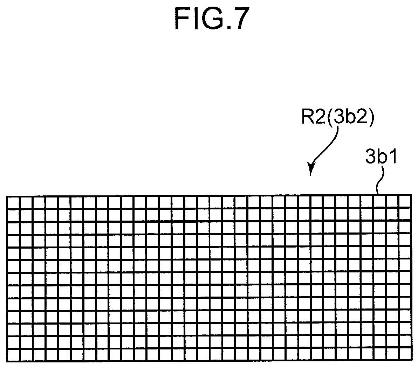

The suction conveying mechanism 3b has a conveying belt 3b2 having a plurality of through holes 3b1 (see FIG. 7) and extending across a plurality of rollers to be along the first conveying path TR1, a suction box 3b3 provided below the conveying belt 3b2, and the suction source 9g connected to the suction box 3b3 to apply the suction force through the through holes 3b1 to the dispersion object A1 on the conveying belt 3b2. In the embodiment, the suction source 9g for the return mechanism 9 serves as the suction source for the suction conveying mechanism 3b, but a suction source different from the suction source 9g may be provided for the suction conveying mechanism 3b. The conveying belt 3b2 has a suction region R2 (whole region of the conveying belt 3b2 in the embodiment: see FIG. 7) preset thereon to draw the dispersion object A1 by suction, the suction region R2 being formed with the through holes 3b1. As shown in FIG. 7, the suction region R2 has a mesh section having a plurality of linear members arranged along two directions crossing each other to define the through holes 3b1. With reference to FIG. 1, the suction box 3b3 is a box member defining a depressurization chamber to be depressurized by the suction source 9g, and has an upper wall 3b31 which the conveying belt 3b2 comes into slidable contact with. The upper wall 3b31 is formed with a plurality of through holes (not shown), and negative pressure in the depressurization chamber is applied to the conveying belt 3b2 through the through holes.

The second conveying part 4 conveys the covering member A2 along the second conveying path TR2 which joins the first conveying path TR1 at a position downstream in a conveying direction of the first conveying part 3 from a predetermined dispersion position P3 of the particles which is preset on the first conveying path TR1. The second conveying path TR2 includes an inclined part extending downward from a position above the first conveying path TR1 and downstream of the rotary body 6 in first conveying path TR1 toward a region between the cylindrical portion 6a and the first conveying path TR1, and a horizontal part extending in the horizontal direction along the first conveying path TR1. The second conveying part 4 has a press roller 4a provided at a boundary between the inclined part and the horizontal part of the second conveying path TR2. Specifically, the press roller 4a is positioned between a region downstream in the conveying direction of the first conveying part 3 from the predetermined dispersion position P3 in the first conveying path TR1 and the outer circumferential surface of the cylindrical portion 6a, and presses the covering member A2 onto the dispersion object A1.

The suction region for the suction conveying mechanism 3b will be described. The suction conveying mechanism 3b is capable of applying the suction force to the dispersion object A1 in a zone including a section from the predetermined dispersion position P3 in the first conveying path TR1 to a press position of the covering member A2 by the press roller 4a. Specifically, the suction box 3b3 is provided along in a section in the first conveying path TR1 from a position upstream from the predetermined dispersion position P3 to a position downstream from the press roller 4a. Thus, the suction force can be applied to the dispersion object A1 from a time before dispersion of the particles to a time after press by the press roller 4a, so that scattering of the dispersed particles can be prevented. The second conveying part 4 may have an adhesive applying member provided at a position upstream from the press roller 4a in the second conveying path TR2 to enable adhesion of the covering member A2 to the dispersion object A1 by the press by the press roller 4a.

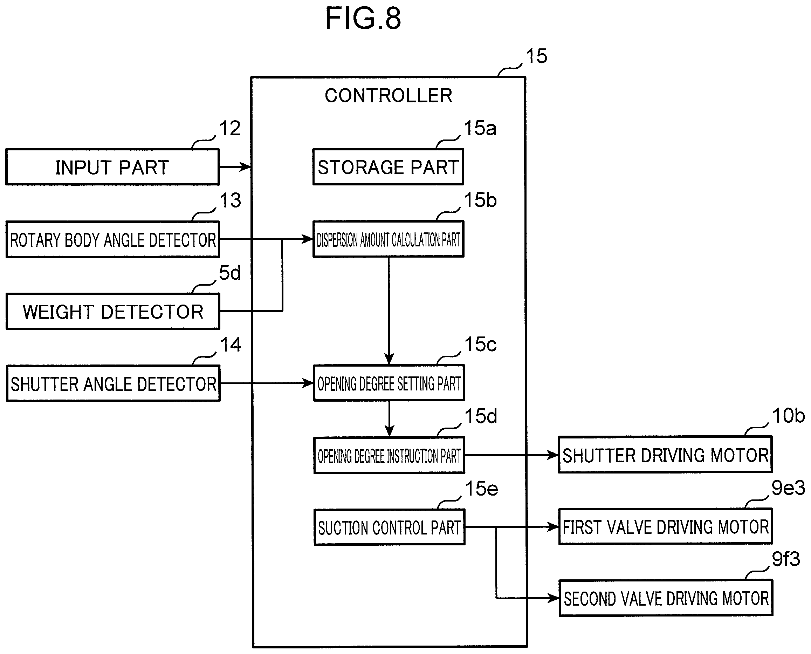

An electrical configuration of the dispersing apparatus body 2 will be described below with reference to FIGS. 3 and 8.

In addition to the configuration described above, the dispersing apparatus body 2 includes an input part 12 that allows an operator to input certain information, a rotary body angle detector 13 that detects a rotation angle of the drive shaft 11b of the rotary body driving motor 11, a shutter angle detector 14 that detects a rotation angle of the drive shaft 10b2 of the shutter driving motor 10b, and a controller 15.

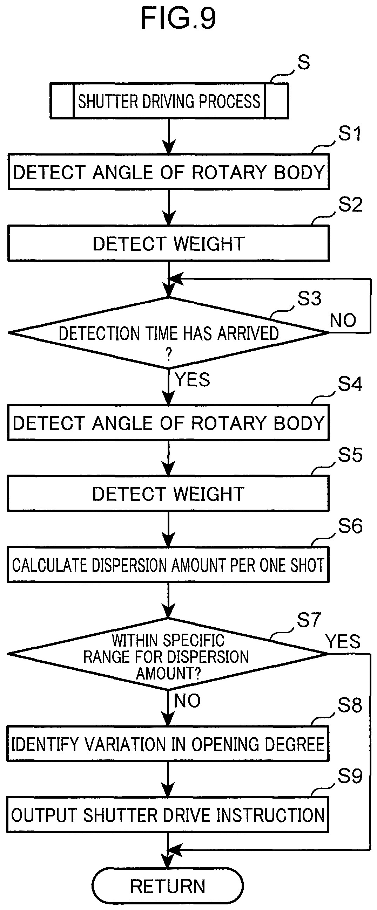

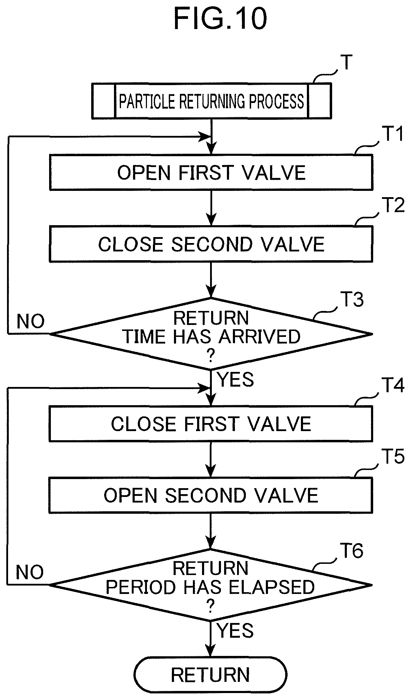

The controller 15 executes a shutter driving process S (see FIG. 9) of controlling drive of the shutter driving motor 10b and a particle returning process T (see FIG. 10) of controlling drive of the first valve driving motor 9e3 and the second valve driving motor 9f3, based on information input from the input part 12, the rotary body angle detector 13, the weight detector 5d, and the shutter angle detector 14. Specifically, the controller 15 has a storage part 15a, a dispersion amount calculation part 15b, an opening degree setting part 15c, an opening degree instruction part 15d, and a suction control part 15e.

The storage part 15a stores an initial setting value and information input by the input part 12. For example, the storage part 15a stores a desired dispersion amount of particles per one shot input by the operator through the input part 12. The one shot indicates a unit of particles to be dispersed through the dispersion holes 6b included in one group G1 shown in FIG. 6.

With reference to FIGS. 3 and 8, the dispersion amount calculation part 15b calculates a dispersion amount of the particles per one shot, based on information from the rotary body angle detector 13, the weight detector 5d, and the storage part 15a. Specifically, the dispersion amount calculation part 15b calculates the number of shots for dispersion during a certain period, based on a rotation angle of the drive shaft 11b of the rotary body driving motor 11 during the certain period detected by the rotary body angle detector 13 and a rotation angle of the drive shaft 11b required for one group G1 to pass by the opening part 7b, which is stored in the storage part 15a. The dispersion amount calculation part 15b calculates the dispersion amount per one shot, based on a decrease in the amount of the particles during the certain period detected by the weight detector 5d and the number of shots for dispersion during the certain period.

The opening degree setting part 15c sets an opening degree of the opening part 7b for reaching a preset desired dispersion amount per one shot, based on the dispersion amount per one shot calculated by the dispersion amount calculation part 15b and a rotation angle of the drive shaft 10b2 of the shutter driving motor 10b detected by the shutter angle detector 14. Specifically, the opening degree setting part 15c determines a deviation between the desired dispersion amount per one shot stored in the storage part 15a and the dispersion amount per one shot calculated by the dispersion amount calculation part 15b. The opening degree setting part 15c determines a rotation angle of the drive shaft 10b2 for causing the dispersion amount per one shot to reach the desired dispersion amount, based on the deviation, the angle of the drive shaft 10b2 detected by the shutter angle detector 14, and a map stored in the storage part 15a. The map indicates a relationship of an estimated variation in the dispersion amount per one shot with respect to the rotation angle of the drive shaft 10b2 of the shutter driving motor 10b.

The opening degree instruction part 15d outputs a drive instruction to the shutter driving motor 10b based on the rotation angle of the drive shaft 10b2 set by the opening degree setting part 15c.

The suction control part 15e opportunely outputs drive instructions to the first valve driving motor 9e3 and the second valve driving motor 9f3 to execute the particle returning process T.

The shutter driving process S executed by the controller 15 will be described below with reference to FIGS. 2 and 9.

The shutter driving process S is started at a certain time (e.g., when a particle dispersion start instruction is input through the input part 12). When the shutter driving process S is started, the rotary body angle detector 13 detects an angle of the rotary body 6 (a rotation angle of the drive shaft 11b) (Step S1).

Next, the weight detector 5d detects a load received by the load receiving plate 5c (see FIG. 2) (Step S2), and arrival of a preset detection time is waited (Step S3).

When it is determined in Step S3 that the detection time has arrived (YES in Step S3), the angle of the rotary body 6 is detected (Step S4), and the weight detector 5d detects the load received by the load receiving plate 5c (Step S5).

Next, a dispersion amount of the particles per one shot is calculated (Step S6). Specifically, in Step S6, a difference between the angles detected respectively in Steps S1 and S4 is obtained, and the number of shots of the particles during a waiting period in Step S3 is calculated based on the difference between the angles. Further, in Step S6, a difference between the loads detected respectively in Steps S2 and S5, i.e., a weight of the dispersed particles is calculated, and the weight is divided by the calculated number of shots to calculate the dispersion amount of the particles per one shot.

Next, in Step S7, it is determined whether the dispersion amount per one shot calculated in step S6 is within a preset specific range for the dispersion amount.

When it is determined in Step S7 that the dispersion amount per one shot calculated in step S6 is within the specific range for the dispersion amount (YES in Step S7), the process returns to Step S1.

On the other hand, when it is determined in Step S7 that the dispersion amount per one shot calculated in Step S6 is not within the specific range for the dispersion amount (NO in Step S7), a variation in the opening degree of the opening part 7b caused by the shutter 10a is identified (Step S8). Specifically, in Step S8, a deviation between the dispersion amount per one shot calculated in Step S6 and the desired dispersion amount per one shot stored in the storage part 15a is identified. Further, in Step S8, a rotation angle of the drive shaft 10b2 for causing the dispersion amount per one shot to reach the desired dispersion amount is identified, based on the deviation, the angle of the drive shaft 10b2 detected by the shutter angle detector 14, and the map stored in the storage part 15a.

Next, an instruction for driving the drive shaft 10b2 based on the variation in the opening degree identified in Step S9 is output to the shutter driving motor 10b (Step S9), and the process returns to Step S1.

Next, the particle returning process T executed by the controller 15 will be described with reference to FIGS. 1 and 10.

The particle returning process T is started at a certain time (e.g., when the particle dispersion start instruction is input through the input part 12). When the particle returning process T is disclosed, the first valve 9e2 is opened (Step T1), and the second valve 9f2 is closed (Step T2). Thus, the insides of the storing container 9b and the delivering container 9c are under negative pressure, so that the particles are drawn into the containers 9b, 9c by suction. On the other hand, the delivering container 9c and the particle introducing member 7 (the outer housing part 7a2) are disconnected from each other, and the particles in the particle introducing member 7 are introduced into the cylindrical portion 6a under their own weight, for the particle introducing member 7 has an unillustrated outside air intake port.

Next, it is determined whether a return time has arrived after the situation as described above lasted for a certain period (Step T3). When it is determined that the return time has not arrived (NO in Step T3), Steps T1 and T2 are executed repeatedly.

On the other hand, when it is determined in Step T3 that the return time has arrived (YES in Step T3), the first valve 9e2 is closed (Step T4), and the second valve 9f2 is opened (Step T5). Thus, the storing container 9b is disconnected from the delivering container 9c, so that the particles are drawn into the storing container 9b by suction. On the other hand, the delivering container 9c and the particle introducing member 7 (the outer housing part 7a2) are connected with each other, so that the particles stored in the delivering container 9c are returned to the particle introducing member 7 under their own weight.

It is then determined whether a return period for returning the particles as described above has elapsed (Step T6). When it is determined that the return period has not elapsed (NO in Step T6), Steps T4 and T5 are executed repeatedly.

On the other hand, when it is determined in Step T6 that the return period has elapsed (YES in Step T6), the process returns to Step T1.

As described above, the edge portion of the opening part 7b comes into contact with the inner circumferential surface of the cylindrical portion 6a while allowing the inner circumferential surface of the cylindrical portion to slide against the edge portion, the edge portion overlapping with the hole formation region R1 in the axial direction entirely so as to confine the particles in cooperation with the cylindrical portion 6a. Therefore, the particles confined by the edge portion of the opening part 7b and the cylindrical portion 6a can be dispersed to the dispersion object A1 with the centrifugal force caused by the rotation of the rotary body 6.

Further, the edge portion of the opening part 7b comes into contact with the region of the inner circumferential surface of the cylindrical portion 6a below the rotational axis C1 while allowing the region of the inner circumferential surface of the cylindrical portion to slide against the edge portion. Accordingly, a length in the circumferential direction at which the axial directional opposite side sections P1, P2 (see FIG. 6) and the inner circumferential surface of the cylindrical portion 6a come into slidable contact with each other is shorter than a length in the circumferential direction at which the lid member and the entire of one circular end surface in the axial direction of the rotary drum come into slidable contact with each other as in the conventional configuration. Therefore, wear of the slidable contact part can be prevented in comparison with the conventional configuration. Consequently, the leakage of the particles from the rotary body 6 can be prevented.

In the embodiment, the edge portion of the opening part 7b and the inner circumferential surface of the cylindrical portion 6a come into slidable contact with each other, with the contact part 7a11, which has an elasticity higher than that of the attached part 7a12, being sandwiched between the attached part 7a12 and the inner circumferential surface of the cylindrical portion 6a. Thus, the contact part deforms elastically according to a condition of the inner circumferential surface of the cylindrical portion 6a, so that wear of the edge portion of the opening part 7b can be prevented.

In the embodiment, the shutter 10a is moved between the contact part 7a11 and the inner circumferential surface of the cylindrical portion 6a by use of the elasticity of the contact part 7a11, so that an opening area of the opening part 7b can be changed to adjust the dispersion amount of the particles.

For example, in a case where the central position CP of the opening part 7b is located on the base line BL extending downward from the rotational axis C1, the particles to be dispersed from the dispersion holes receive a force in the horizontal direction as a centrifugal force by the rotary body 6. In this case, the centrifugal force cannot be effectively utilized to proactively place the particles on the dispersion object located below the rotary body 6. On the other hand, in a case as in the embodiment where the central position CP of the opening part 7b is located upstream in the rotational direction Y1 of the rotary body from the base line BL extending downward from the rotational axis in a side view, a downward component F1 can be given with respect to a direction of the centrifugal force applied to the particles in a direction due to the rotation of the rotary body 6. Thus, the centrifugal force can be effectively utilized to place the particles on the dispersion object A1.

Normally, the particles are dispersed to the dispersion object A1 through the dispersion holes 6b, but may adhere to the inner circumferential surface of the cylindrical portion 6a due to, e.g., static electricity. In this case, the particles adhering to and remaining on the cylindrical portion 6a increase an apparent thickness of the cylindrical portion 6a. Therefore, sliding resistance between the inner circumferential surface of the cylindrical portion 6a and the edge portion of the opening part 7b is increased, and thus wear of the edge portion of the opening part 7b may be accelerated. In the embodiment, however, the particles adhering to and remaining on the inner circumferential surface of the cylindrical portion 6a due to, e.g., the static electricity can be removed by the scraper 8, so that the acceleration of the wear of the edge portion of the opening part 7b as described above can be prevented.

In the embodiment, the particles removed by the scraper 8 can be returned to the particle introducing member 7, and reintroduced into the cylindrical portion 6a through the opening part 7b.

Since the embodiment involves the press roller 4a that presses the covering member A2 onto the dispersion object A1, the dispersion object A1 through the dispersion of the particles is covered by the covering member A2, so that the scattering of the particles from the dispersion object A1 can be prevented. The press roller 4a is positioned between a region downstream in the conveying direction of the first conveying part 3 from the predetermined dispersion position P3 in the first conveying path and the outer circumferential surface of the cylindrical portion 6a, i.e., positioned close to the predetermined dispersion position P3. Therefore, the dispersion object A1 can be covered by the covering member A2 immediately after the dispersion of the particles.

Since the embodiment involves the suction conveying mechanism 3b, the scattering of the particles between the predetermined dispersion position P3 and where the dispersion object A1 is covered by the covering member A2 can be prevented.

In the embodiment, the suction region R2 has the mesh section. Therefore, the opening area of the through holes can be reduced in comparison with a suction region having a through hole formed in a belt by a process such as punching. This can prevent a mark of the through holes caused by the suction force from remaining on a dispersion object A1 made of a flexible material (e.g., nonwoven fabric).

The present invention is not limited to the embodiment; for example, the present invention may be configured as follows.

In the embodiment, the edge portion of the opening part has the contact part 7a11 and the attached part 7a12. In this regard, the contact part 7a11 may be omitted, and the attached part 7a12 and the inner circumferential surface of the cylindrical portion 6a may come into direct slidable contact with each other.

The embodiment involves the shutter 10a, but the shutter 10a may be omitted. In this case, an adjustment for the opening part can be made by, e.g., reducing the opening area of the opening part 7b by placing a baffle plate in the particle introducing member 7 while the dispersing apparatus body 2 is stopped.

The embodiment involves the shutter driving motor 10b that drives the shutter 10a rotatably. In this regard, the shutter driving motor 10b may be omitted and the shutter 10a may be manually rotated.

In the embodiment, the particle introducing member 7 is attached to the support member 5 such that the central position CP of the opening part 7b is located upstream in the rotational direction Y1 from the base line BL, but the central position CP of the opening part 7b is not limited to the position described above. For example, the central position CP of the opening part 7b may be located on the base line BL, and/or the central position CP of the opening part 7b may be located downstream in the rotational direction Y1 from the base line BL. In this case, the first conveying path TR1 of the first conveying part 3 can be adjusted such that a direction in which a centrifugal force by the rotary body 6 acts intersects with the dispersion object A1 to utilize the centrifugal force as a force toward the dispersion object A1.

In the embodiment, the removal member is exemplified by the scraper 8 in shape of a plate (spatula) that has the base end part 8a, the leading end part 8b, and the middle part 8c. However, the removal member is not limited to the scraper 8, as long as it removes the particles remaining on the inner circumferential surface of the cylindrical portion 6a. For example, a sponge member (e.g., melamine sponge) or a fabric member (e.g., microfiber) is available as the removal member.

The embodiment involves the scraper 8 and the return mechanism 9, but these may be omitted. In this case, provision of means for removing static electricity on the cylindrical portion 6a enables prevention of adhesion of the particles to the inner circumferential surface of the cylindrical portion 6a.

The embodiment involves the first conveying part 3 and the second conveying part 4, but at least one of the conveying parts 3, 4 may be omitted. In this case, the at least one of the conveying parts 3, 4 can serve as a separate unit that can be used in combination with the dispersing apparatus body 2.

In the embodiment, the suction conveying mechanism 3b of the first conveying part 3 utilizes the suction force by the suction source 9g to hold the particles on the dispersion object A1, but the means for holding the particles is not limited to the suction force. For example, in a case where a nonwoven fabric is used as the dispersion object A1, raising a surface of the dispersion object A1 at a position upstream from the predetermined dispersion position P3 enables the particles dispersed thereafter to be held on the dispersion object.

The embodiment involves the conveying belt 3b2 having the mesh section. In this regard, a conveying belt having a through hole formed by a process such as punching may be used. In this case, use of a dispersion object with lower flexibility enables prevention of a mark of the through hole on a lower surface of the dispersion object caused by suction.

The specific embodiment described above mainly involves inventions having the following configurations.

To solve the problem as described above, provided is a first invention directed to a particle dispersing apparatus to disperse particles to a dispersion object, including: a support member; a rotary body having a cylindrical portion that is supported by the support member rotatably around a rotational axis extending in a horizontal direction and surrounds the rotational axis, and a dispersion hole extending through the cylindrical portion in a hole formation region that is a part of the cylindrical portion in an axial direction along the rotational axis; and a particle introducing member that has a housing portion having a housing space for housing the particles and an opening part formed in a lower end of the housing portion to introduce the particles into the cylindrical portion, and is attached to the support member such that the opening part is located in the cylindrical portion, and in the particle dispersing apparatus, the opening part of the housing portion has an edge portion that comes into contact with a region of an inner circumferential surface of the cylindrical portion below the rotational axis while allowing the region of the inner circumferential surface of the cylindrical portion to slide against the edge portion, the edge portion overlapping with the hole formation region in the axial direction entirely so as to confine the particles in cooperation with the cylindrical portion.

In the first invention, the edge portion of the opening part comes into contact with the inner circumferential surface of the cylindrical portion while allowing the inner circumferential surface of the cylindrical portion to slide against the edge portion, the edge portion overlapping with the hole formation region in the axial direction entirely so as to confine the particles in cooperation with the cylindrical portion. Therefore, the particles confined by the edge portion of the opening part and the cylindrical portion can be dispersed to the dispersion object with the centrifugal force caused by the rotation of the rotary body.

In order that the edge portion of the opening part "confines the particles in cooperation with the cylindrical portion", two sections (hereinafter, referred to as axial directional opposite side sections) of the edge portion of the opening part, which are across the hole formation region in the axial direction, are required to come into contact with the inner circumferential surface of the cylindrical portion such that the inner circumferential surface is slidable so as to prevent leakage of the particles in the axial direction. On the other hand, even if particles leak from a region between the axial directional opposite side sections of the edge portion of the opening part on the inner circumferential surface of the cylindrical portion, the particles are dispersed from the dispersion holes due to a centrifugal force caused by the rotation of the cylindrical portion, thus resulting in no substantial leakage of the particles. In other words, the axial directional opposite side sections of the edge portion of the opening part are the most important to "confine the particles in cooperation with the cylindrical portion".

In this regard, in the first invention, the edge portion of the opening part comes into contact with the region of the inner circumferential surface of the cylindrical portion below the rotational axis while allowing the region of the inner circumferential surface of the cylindrical portion to slide against the edge portion. Accordingly, a length in the circumferential direction at which the axial directional opposite side sections and the inner circumferential surface of the cylindrical portion come into slidable contact with each other is shorter than a length in the circumferential direction at which the lid member and the entire of one circular end surface in the axial direction of the rotary drum come into slidable contact with each other as in the conventional configuration. Therefore, wear of the slidable contact part can be prevented in comparison with the conventional configuration. Consequently, the leakage of the particles from the rotary body can be prevented.

According to a second invention, in the first invention, preferably, the edge portion of the opening part has a contact part that comes into contact with the inner circumferential surface of the cylindrical portion, and an attached part that is attached to the contact part, the attached part and the inner circumferential surface of the cylindrical portion sandwiching the contact part, and the contact part has an elasticity higher than an elasticity of the attached part.

In the second invention, the edge portion of the opening part and the inner circumferential surface of the cylindrical portion come into slidable contact with each other, with the contact part, which has an elasticity higher than that of the attached part, being sandwiched between the attached part and the inner circumferential surface of the cylindrical portion. Thus, the contact part deforms elastically according to a condition of the inner circumferential surface of the cylindrical portion, so that wear of the edge portion of the opening part can be prevented.

According to a third invention, preferably, the second invention further includes an opening degree adjusting member that is attached to the support member so as to move between the contact part and the inner circumferential surface of the cylindrical portion while causing the contact part to deform elastically to adjust an opening degree of the opening part of the particle introducing member.

In the third invention, the opening degree adjusting member is moved between the contact part and the inner circumferential surface of the cylindrical portion by use of the elasticity of the contact part, so that an opening area of the opening part can be changed to adjust the dispersion amount of the particles.

According to a fourth invention, in any one of the first to third inventions, preferably, the particle introducing member is attached to the support member such that a central position of the opening part is located upstream in a rotational direction of the rotary body from a base line extending downward from the rotational axis, in a side view of the rotary body along the rotational axis.

For example, in a case where the central position of the opening part is located on the base line extending downward from the rotational axis, the particles to be dispersed from the dispersion holes receive a force in the horizontal direction as a centrifugal force by the rotary body. In this case, the centrifugal force cannot be effectively utilized to proactively place the particles on the dispersion object located below the rotary body. On the other hand, in a case as in the fourth invention where the central position of the opening part is located upstream in the rotational direction of the rotary body from the base line extending downward from the rotational axis in a side view, a downward component can be given to the centrifugal force applied to the particles in a direction due to the rotation of the rotary body. Thus, the centrifugal force can be effectively utilized to place the particles on the dispersion object.

According to a fifth invention, preferably, any one of the first to fourth inventions further includes a removal member that is attached to the support member so as to come into contact with a region of the inner circumferential surface of the cylindrical portion located below the rotational axis and downstream in a rotational direction of the rotary body from the opening part while allowing the region of the inner circumferential surface of the cylindrical portion to slide against the removal member to remove particles remaining on the inner circumferential surface of the cylindrical portion.

Normally, the particles are dispersed to the dispersion object through the dispersion holes, but may adhere to the inner circumferential surface of the cylindrical portion due to, e.g., static electricity. In this case, the particles remaining on the cylindrical portion increase an apparent thickness of the cylindrical portion. Therefore, sliding resistance between the inner circumferential surface of the cylindrical portion and the edge portion of the opening part is increased, and thus wear of the edge portion of the opening part may be accelerated. In this regard, in the fifth invention, the particles adhering to and remaining on the inner circumferential surface of the cylindrical portion due to, e.g., the static electricity can be removed by the removal member, so that the acceleration of the wear of the edge portion of the opening part as described above can be prevented.

According to a sixth invention, preferably, the fifth invention further includes a return mechanism that returns the particles removed by the removal member to the particle introducing member.

In the sixth invention, the particles removed by the removal member can be returned to the particle introducing member, and reintroduced into the cylindrical portion through the opening part.

According to a seventh invention, preferably, any one of the first to sixth inventions further includes a first conveying part that conveys the dispersion object along a first conveying path which is set below the cylindrical portion; and a second conveying part that conveys a covering member along a second conveying path which joins the first conveying path at a position downstream in a conveying direction of the first conveying part from a predetermined dispersion position of the particles which is preset on the first conveying path, and the second conveying part has a press roller that presses the covering member onto the dispersion object, the press roller being positioned between a region downstream in the conveying direction of the first conveying part from the predetermined dispersion position in the first conveying path and an outer circumferential surface of the cylindrical portion.

Since the seventh invention has the press roller that presses the covering member onto the dispersion object, the dispersion object through the dispersion of the particles is covered by the covering member, so that the scattering of the particles from the dispersion object can be prevented. The press roller is positioned between a region downstream in the conveying direction of the first conveying part from the predetermined dispersion position in the first conveying path and the outer circumferential surface of the cylindrical portion, i.e., positioned close to the predetermined dispersion position. Therefore, the dispersion object can be covered by the covering member immediately after the dispersion of the particles.

According to an eighth invention, in the seventh invention, preferably, the first conveying part has a suction conveying mechanism that conveys the dispersion object while applying a suction force to the dispersion object in a section from the predetermined dispersion position in the first conveying path to a press position of the covering member by the press roller.

The eighth invention involves the suction conveying mechanism, so that the scattering of the particles between the predetermined dispersion position and where the dispersion object is covered by the covering member can be prevented.

According to a ninth invention, in the eighth invention, preferably, the suction conveying mechanism includes a conveying belt having a plurality of through holes formed in a suction region preset to draw the dispersion object by suction, and a suction source to apply the suction force to the dispersion object on the conveying belt through the through holes, and the suction region has a mesh section including a plurality of linear members arranged along two directions crossing each other to define the through holes.

In the ninth invention, the suction region has the mesh section. Therefore, the opening area of the through holes can be reduced in comparison with a suction region having a through hole formed in a belt by a process such as punching. This can prevent a mark of the through holes caused by the suction force from remaining on a dispersion object made of a flexible material (e.g., nonwoven fabric).

## Claims

1. A particle dispersing apparatus to disperse particles to a dispersion object, comprising:
a support member;
a rotary body having a cylindrical portion that is supported by the support member rotatably around a rotational axis extending in a horizontal direction and surrounds the rotational axis, and a dispersion hole extending through the cylindrical portion in a hole formation region that is a part of the cylindrical portion in an axial direction along the rotational axis; and
a particle introducing member that has a housing portion having a housing space for housing the particles and an opening part formed in a lower end of the housing portion to introduce the particles into the cylindrical portion, and is attached to the support member such that the opening part is located in the cylindrical portion, wherein
the opening part of the housing portion has an edge portion that comes into contact with a region of an inner circumferential surface of the cylindrical portion below the rotational axis while allowing the region of the inner circumferential surface of the cylindrical portion to slide against the edge portion, the edge portion overlapping with the hole formation region in the axial direction entirely so as to confine the particles in cooperation with the cylindrical portion.

2. The particle dispersing apparatus according to claim 1, wherein
the edge portion of the opening part has a contact part that comes into contact with the inner circumferential surface of the cylindrical portion, and an attached part that is attached to the contact part, the attached part and the inner circumferential surface of the cylindrical portion sandwiching the contact part, and
the contact part has an elasticity higher than an elasticity of the attached part.

3. The particle dispersing apparatus according to claim 2, further comprising:
an opening degree adjusting member that is attached to the support member so as to move between the contact part and the inner circumferential surface of the cylindrical portion while causing the contact part to deform elastically to adjust an opening degree of the opening part of the particle introducing member.

4. The particle dispersing apparatus according to any one of claims 1 to 3, wherein the particle introducing member is attached to the support member such that a central position of the opening part is located upstream in a rotational direction of the rotary body from a base line extending downward from the rotational axis, in a side view of the rotary body along the rotational axis.

5. The particle dispersing apparatus according to any one of claims 1 to 4, further comprising:
a removal member that is attached to the support member so as to come into contact with a region of the inner circumferential surface of the cylindrical portion located below the rotational axis and downstream in a rotational direction of the rotary body from the opening part while allowing the region of the inner circumferential surface of the cylindrical portion to slide against the removal member to remove particles remaining on the inner circumferential surface of the cylindrical portion.

6. The particle dispersing apparatus according to claim 5, further comprising:
a return mechanism that returns the particles removed by the removal member to the particle introducing member.

7. The particle dispersing apparatus according to any one of claims 1 to 6, further comprising:
a first conveying part that conveys the dispersion object along a first conveying path which is set below the cylindrical portion; and
a second conveying part that conveys a covering member along a second conveying path which joins the first conveying path at a position downstream in a conveying direction of the first conveying part from a predetermined dispersion position of the particles which is preset on the first conveying path, wherein
the second conveying part has a press roller that presses the covering member onto the dispersion object, the press roller being positioned between a region downstream in the conveying direction of the first conveying part from the predetermined dispersion position in the first conveying path and an outer circumferential surface of the cylindrical portion.

8. The particle dispersing apparatus according to claim 7, wherein the first conveying part has a suction conveying mechanism that conveys the dispersion object while applying a suction force to the dispersion object in a section from the predetermined dispersion position in the first conveying path to a press position of the covering member by the press roller.

9. The particle dispersing apparatus according to claim 8, wherein
the suction conveying mechanism includes a conveying belt having a plurality of through holes formed in a suction region preset to draw the dispersion object by suction, and a suction source to apply the suction force to the dispersion object on the conveying belt through the through holes, and
the suction region has a mesh section including a plurality of linear members arranged along two directions crossing each other to define the through holes.
